# EUROPEAN PATENT APPLICATION

(11) **EP 2 687 594 A1**
(43) Date of publication of application: **22.01.2014**
(21) Application number: 12757775.7
(22) Date of filing: 16.03.2012
(51) Int. Cl.: C12N 5/077, C12Q 1/02, C12M 3/00

(54) **CULTURE METHOD, GROUP OF MATURE ADIPOCYTES, AND DRUG SCREENING METHOD**

(30) Priority: 16.03.2011 JP 2011057770
(71) Applicant: Kuraray Co., Ltd., Okayama 710-0801 (JP)
(72) Inventor: ITCHODA, Yoko, Tsukuba-shi Ibaraki 305-0841 (JP); HOSODA, Masaya, Tsukuba-shi Ibaraki 305-0841 (JP); FUKUDA, Motohiro, Tainai-shi Niigata 959-2691 (JP); NAGAYAMA, Masafumi, Sapporo-shi Hokkaido 060-0808 (JP)
(74) Representative: Müller-Boré & Partner Patentanwälte PartG mbB
(86) International application number: PCT/JP2012/001872
(87) International publication number: WO 2012/124353

(57) **Abstract**

In a culture method for adipocytes, seeded adipocytes are cultured while being allowed to adhere to a culture bottom surface (14) and walls (12) disposed perpendicularly to the culture bottom surface (14), without suspending the adipocytes in a culture solution, thereby obtaining mature adipocytes in which spherical lipid droplets having increased in size are generated within cells. This method uses a culture chamber (10) in which the walls (12) are formed perpendicular to the culture bottom surface (14). Spherical mature adipocytes containing spherical lipid droplets having increased in size within cells are cultured in a state where a cell or an aggregate of cells is allowed to adhere to two or more locations on the walls (12) and the culture bottom surface (14), thereby obtaining adipocytes having a shape similar to that of adipocytes in vivo.

## Description

### Technical Field

The present invention relates to culture of adipocytes, and more particularly, to a culture method for culturing adipocytes containing spherical lipid droplets within cells to make it easier to use the adipocytes for testing and the like, a group of mature adipocytes, and a drug screening method.

### Background Art

In recent years, it has become apparent that adipose tissue is an important organ not only for storage and release of nutrients, but also for release of various bioactive substances such as leptin. It has been recognized also as an important organ that causes diabetes and various adult diseases. Therefore, adipocytes have been studied as a target of drugs to be provided for treatments of these diseases. The use of cultured adipocytes is essential for development of such drugs and screening tests thereof.

As for preadipocytes or progenitor cells of fibroblast-like cells in adipose tissue in vivo, minute lipid droplets are first formed within cells. Lipid droplets that are gradually stored and increased in size are stored and form spherical mature adipocytes (mature adipocytes). The process of differentiation of preadipocytes into mature adipocytes is important for formation of adipocyte tissue. Accordingly, it is significant to examine the effects of drugs during this process.

For example, Patent Literature 1 discloses a culture medium for culturing and assaying visceral adipocytes under conditions more similar to the in vivo conditions by using a monolayer cell line. However, in the monolayer culture method using a monolayer cell line, it is difficult to promote the differentiation induction until fully mature adipocytes are obtained. Specifically, at early stages, adipocytes are cultured while allowing them to adhere to a flask bottom surface. When the adipocytes have increased in size while storing therein oil droplets due to the differentiation of the adipocytes, the form of the adipocytes is changed into a round shape, so that the adipocytes float. For this reason, mature adipocytes are easily peeled off from an adhesive surface of a flask. As a result, only the cells in the intermediate stage of the differentiation in which oil droplets are stored in the adipocytes in a multilocular manner can be observed. Thus, it has been difficult to observe the differentiation and culture for a long period of time and final differentiation, or to conduct experiments on physiologically active substances and the like which are released only from mature adipocytes.

As a method for solving such a problem, Patent Literature 2 discloses a culture method for unilocular adipocytes. The adipocytes are suspended in a culture solution due to neutral fat contained in the cytoplasm. The culture method for unilocular adipocytes disclosed in Patent Literature 2 discloses a technique using a method (ceiling culture) for culturing adipocytes while allowing them to adhere to an inside upper surface (ceiling surface) of a flask which is fully filled with a culture medium. When ceiling culture of unilocular adipocytes is carried out for several days by use of this method, most of the cells cause a part of the cytoplasm to stretch or dilate to firmly adhere to the ceiling surface of the flask, so that the adipocytes change in form into mature adipocytes containing various sizes of lipid droplets in the vicinity of large lipid droplets.

According to Non Patent Literatures 1 and 2, the size of an aggregate, which is an aggregate of two or more cells, has been studied.

### Citation List

### Patent Literature

[Patent Literature 1] International Patent Publication No. WO 2007/125859
[Patent Literature 2] Japanese Unexamined Patent Application Publication No. 2000-83656

### Non Patent Literature

[Non Patent Literature 1] Rachel Glicklis et al., "Modeling Mass Transfer in Hepatocyte Spheroids via Cell Viability, Spheroid Size, and Hepatocellualr Functions", Published online in Wiley InterScience (www.interscience.wiley.com), 23 April, 2004
[Non Patent Literature 2] Franziska Hirschhaeuser et al., "Multicelluar tumor spheroids: An underestimated tool is catching up again", Journal of Biotechnology 148, 2010, pp. 3-15

### Summary of Invention

### Technical Problem

In screening tests, however, the amount, concentration, or the like of the drug to be evaluated is varied under the same conditions, and the effects of the drug are measured. Accordingly, it is necessary to use culture chambers which are made of the same material and have the same shape, for example. Examples of the culture chambers include a plastic petri dish, a glass petri dish, and a well plate (microplate). Examples of the well plate include normalized 6-well, 12-well, 48-well, 96-well, and 384-well plates. Furthermore, to meet the specifications of such culture chambers, an automatic culture device, an automatic analyzer, or an evaluation device capable of simultaneously measuring multiple specimens has been developed. Accordingly, in the industrial field, there is a demand for a cell culture method using a culture chamber which has the same shape and the same operation as those in the specifications of such culture chambers and which has high transparency in terms of observation performance. On the other hand, the technique of Patent Literature 2 in which adipocytes are cultured on the ceiling surface needs to use a culture chamber having a form which is significantly different from that of various standard culture chambers. This results in a problem that the culture chamber for use in ceiling culture cannot be used in an analyzer and a culture device which use a normalized well plate. There is another problem that the culture chamber for use in ceiling culture cannot be used in a measurement device which uses a normalized well plate, with the result that a screening test cannot be conducted using such a measurement device.

In view of the above, it is an object of the present invention to provide a cell culture method for culturing adipocytes to make it easier to use the adipocytes for testing and the like, by use of a culture chamber including a culture bottom surface and walls disposed perpendicularly to the culture bottom surface. More specifically, it is an object of the present invention to provide a cell culture method for culturing adipocytes by use of a culture chamber which is replaceable with a well plate (normalized well plate) or the like which is applicable to conventionally-used devices for culture, testing, and evaluation.

### Solution to Problem

An aspect of a culture method for adipocytes according to the present invention is a culture method for adipocytes which cultures seeded adipocytes while allowing the adipocytes to adhere to a culture bottom surface and walls disposed perpendicularly to the culture bottom surface, to obtain spherical mature adipocytes in which spherical lipid droplets having increased in size are generated within cells, without suspending the adipocytes in a culture solution. The culture method uses a culture chamber in which the walls are formed perpendicularly to the culture bottom surface. The culture method obtains spherical mature adipocytes which contain spherical lipid droplets within cells and in which lipid droplets having a three-dimensional shape have increased in size, in a state where a cell or a part of an aggregate, which is an aggregate of two or more cells, is allowed to adhere to two or more locations on the walls and the culture bottom surface.

In one aspect of the culture method for adipocytes according to the present invention, it is preferable that the lipid droplets have a volume in the range from 0.5×10³ µm³ to 3×10⁶ µm³, or that a value obtained by dividing a minimum diameter (minor axis) of the lipid droplets by a maximum diameter (major axis) thereof be in the range from 0.4 to 1.0.

As for the size the aggregate, which is an aggregate of two or more cells, according to the present invention, it is preferable, in view of Patent Literatures 1 and 2, that an aggregate having such a size that a distance from an outermost layer adhering to a culture medium is equal to or less than 250 µm be used so as to supply nutrients and oxygen to the center of the aggregate and allow the aggregate to survive. To keep the state of each cell within each well constant, an aggregate having a spherical or hemispherical shape with a uniform diameter of 500 µm or less is more preferably used.

The surface of the culture chamber including the walls perpendicular to the culture bottom surface is preferably subjected to a plasma treatment or coated with an inorganic material, thereby being subjected to a hydrophilic treatment so as to enhance the adhesion property of cell adhesive protein (collagen, etc.). Furthermore, the surface of the culture chamber including the culture bottom surface and the walls disposed perpendicularly to the culture bottom surface is preferably coated with an extracellular matrix as typified by collagen or fibronectin, or is preferably coated with a synthetic material.

### Advantageous Effects of Invention

According to the present invention, it is possible to culture adipocytes in a form replaceable with a well plate or the like, which is especially applicable to conventionally-used devices for culture, testing, and evaluation, to make it easier to use the adipocytes for testing and the like, by use of a culture chamber including a culture bottom surface and walls disposed perpendicularly to the culture bottom surface.

### Brief Description of Drawings

Fig. 1 is a plan view showing a structure of a culture chamber according to an embodiment of the present invention;
Fig. 2 is a sectional view taken along the line II-II of the culture chamber shown in Fig. 1;
Fig. 3A is a schematic view showing a state where cells are cultured using the culture chamber shown in Fig. 1;
Fig. 3B is a diagram showing a state where one adipocyte is cultured in each partitioned region;
Fig. 3C is a diagram showing a state where the adipocytes shown in Fig. 3B are increased in size;
Fig. 4 is a plan view showing another structure of the culture chamber according to an embodiment of the present invention;
Fig. 5 is a sectional view taken along the line V-V of the culture chamber shown in Fig. 4;
Fig. 6A is a plan view showing further another structural example of the culture chamber according to an embodiment of the present invention;
Fig. 6B is a schematic view showing a state where adipocytes are cultured in the culture chamber shown in Fig. 6A, when viewed from above;
Fig. 6C is a sectional view taken along the line VI-VI of Fig. 6B;
Fig. 7A is a plan view showing further another structural example of the culture chamber according to an embodiment of the present invention;
Fig. 7B is a sectional view taken along the line VII-VII of Fig. 7A;
Fig. 8 is a plan view showing further another structure of the culture chamber according to an embodiment of the present invention;
Fig. 9 is a sectional view taken along the line IX-IX of the culture chamber shown in Fig. 8;
Fig. 10 is a phase-contrast microphotograph of cells after 20 days of differentiation in Comparative Example 1 using a 40x objective lens;
Fig. 11 is a phase-contrast microphotograph of cells after 20 days of differentiation in Example 1 using a 40x objective lens;
Fig. 12 is a phase-contrast microphotograph of cells after 20 days of differentiation in Example 2 using a 40x objective lens;
Fig. 13A is a photograph obtained by capturing a slice image of stained cells in Example 2 (bottom: 0 µm in Z-axis direction);
Fig. 13B is a photograph obtained by capturing a slice image of stained cells in Example 2 (10 µm in Z-axis direction);
Fig. 13C is a photograph obtained by capturing a slice image of stained cells in Example 2 (20 µm in Z-axis direction);
Fig. 13D is a photograph obtained by capturing a slice image of stained cells in Example 2 (30 µm in Z-axis direction);
Fig. 13E is a photograph obtained by capturing a slice image of stained cells in Example 2 (40 µm in Z-axis direction);
Fig. 13F is a photograph obtained by capturing a slice image of stained cells in Example 2 (50 µm in Z-axis direction); and
Fig. 13G is a photograph obtained by capturing a slice image of stained cells in Example 2 (upper portion: 60 µm in Z-axis direction).

### Description of Embodiments

Hereinafter, an embodiment of the present invention will be described with reference to the drawings. However, the present invention is not limited to the embodiment described below. For clarity of explanation, omission and simplification are made as appropriate in the following description and the drawings. The components having the same structure or function and corresponding parts in the drawings are denoted by the same reference numerals, and the description thereof is omitted.

### First Embodiment

According to a first embodiment, a cell culture method for culturing adipocytes to make it easier to use the adipocytes for testing and the like is achieved in the following manner, by use of a culture chamber including a culture bottom surface and walls disposed perpendicularly to the culture bottom surface. (1) Adipocytes are cultured in forms similar to mature adipocytes existing in a living body, without suspending the adipocytes in a culture solution. Specifically, the adipocytes are cultured in a state where spherical lipid droplets increased in size are contained in the cells. (2) A culture chamber which can be easily used for testing and the like is used.

Specifically, adipose tissue in vivo presents a state where spherical lipid droplets are stored within a cell membrane of one adipocytes and a plurality of spherical adipocytes aggregates. This embodiment achieves a cell culture method for obtaining mature adipocytes having a shape more similar to that of the adipocytes in vivo. For example, adipocytes for use in experiments have a shape close to a two-dimensional state, rather than a spherical shape, so as to make the adipocytes grow while allowing them to adhere to a bottom of a plate, a flask, or the like. At the stage of maturation, that is, when the ratio of the volume of lipid droplets present in the cell membrane increases, cells are removed from the culture chamber due to the buoyancy of the lipid droplets. This makes it impossible to culture the cells and makes it difficult to obtain mature adipocytes. Thus, there is a large difference in form among adipocytes, which causes a problem that substances and the like which are originally secreted from the adipocytes in vivo are different from those ex vivo. In view of this, according to this embodiment, adipocytes are cultured while allowing them to adhere to both the culture bottom surface and the walls perpendicular to the culture bottom surface, thereby making it possible to obtain mature adipocytes or a cell mass having a shape more similar to a spherical shape, as compared with the conventional culture method, in a state where the adipocytes are three-dimensionally arranged and are allowed to adhere to the culture chamber.

Moreover, the culture of adipocytes is achieved in a state where the adipocytes are applicable to a culture chamber for use in testing, for example, a screening test, or development of drugs, and to instruments (microscope, etc.) for observation. For example, the use of plastic, which is a transparent material, to enable the use for observation with an existing microscope enables observation using a chamber for culture, without the need for transferring cells to another chamber.

In the following description, a culture chamber according to the first embodiment will be described first, and thereafter, a cell culture method will be described.

### 1. Culture Chamber

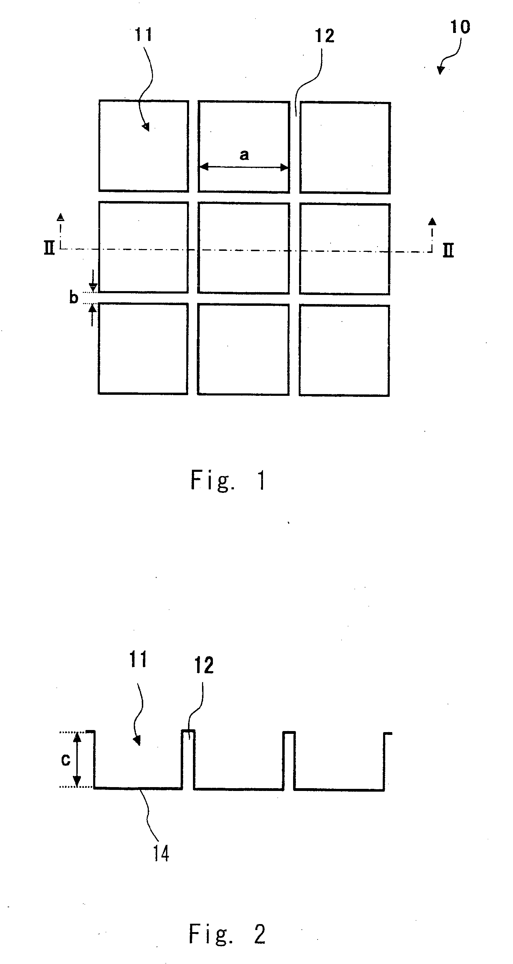

Fig. 1 is a plan view showing a structure of a culture chamber according to this embodiment. Fig. 2 is a sectional view taken along the line II-II of Fig. 1. As shown in Fig. 1, a culture chamber 10 includes walls 12 which are perpendicular to a culture bottom surface 14 (hereinafter, the perpendicular walls are referred to also as "walls", as needed). Adipocytes are cultured while allowing them to adhere to the culture bottom surface 14 and the walls 12. Accordingly, the vicinity of an intersection between the culture bottom surface 14 and each wall 12 corresponds to a cell culture position. The culture chamber 10 preferably includes a plurality of cell culture positions where the adipocytes can adhere. The culture bottom surface 14 (culture surface) herein described indicates a portion serving as a bottom of a space in which cells are cultured (a bottom of a culture space).

Referring to Fig. 1, in the culture chamber 10, the culture bottom surface 14 includes partitioned regions (culture regions) 11 which are partitioned by the perpendicularly-formed walls 12. Fig. 1 shows a distance "a" between the facing walls 12 (a distance between the opposing walls), and a width "b" and a height "c" of each wall 12. Each space formed by the partitioned regions 11 and the walls 12 corresponds to a cell culture position (space for culture).

The shape of each of the walls 12 which are formed perpendicularly to the culture bottom surface 14 is not particularly limited. For example, the walls may be continuous or non-continuous walls. Alternatively, the walls may have a planar shape or a curved shape. More alternatively, the walls may have a polygonal shape or a columnar shape. The shape of each wall is not particularly limited as long as each wall is perpendicular to the culture bottom surface 14. To reproduce the functions of adipocytes, the culture chamber 10 preferably includes the regions 11 partitioned by the walls 12 so that one to several tens of cells in which lipid droplets are stored can exist at a constant location.

The distance "a" between the facing walls 12 is preferably 70 µm to 300 µm. The width "b" of each wall 12 is not particularly limited, but is preferably 20 µm or less, and more preferably, 15 µm or less, so that the cells are prevented from adhering to each wall 12.

The height "c" of each wall 12 is not particularly limited as long as the cells to be cultured in the regions 11, each of which is partitioned by the walls 12, are prevented from running on the walls 12 and moving to the adjacent regions (regions 11 partitioned by the walls 12). The height "c" is preferably 50 µm to 500 µm. In the case of forming adipocytes containing lipid droplets having an equivalent diameter of 70 µm, for example, the height "c" of each wall 12 is preferably 50 µm to 150 µm. In the case of forming adipocytes having an equivalent diameter of 150 µm, for example, the height "c" is preferably 50 µm to 300 µm.

The walls 12 perpendicular to the culture bottom surface 14 only need to be substantially perpendicular to the culture bottom surface 14. For example, the angle formed between the culture bottom surface 14 and each wall 12 (the angle formed therebetween is an angle on the opposite side of the partitioned region 11 to which adipocytes adhere) may be in the range from 88° to 90°, or in the range from 85° to 90°.

The angle formed between the culture bottom surface 14 and each wall 12 needs to be an angle at which the cells are prevented from running on the walls. Accordingly, in a portion equal to or more than 50% from the top of a side surface, the angle is preferably 80° to 90°, and more preferably, 85° to 90°.

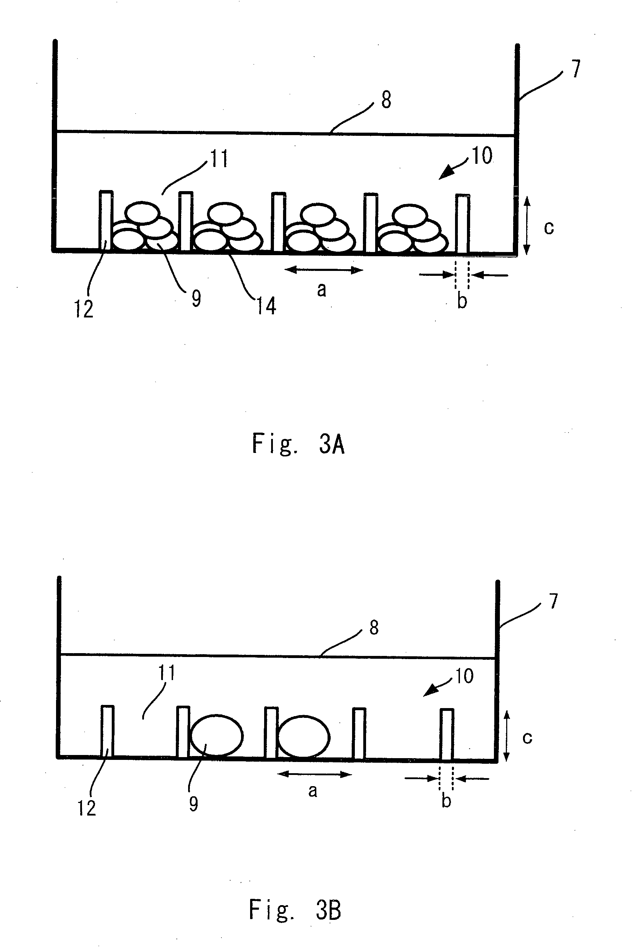

Fig. 3A is a schematic view showing a state where cells are cultured using the culture chamber shown in Fig. 1. Fig. 3A is a perspective view when viewed from a side of the culture chamber. The culture chamber 10 is filled with a culture medium 8. The figure shows a state where the adipocytes 9 form a cell mass and are cultured in each of the partition regions 11 partitioned by the walls 12. Figs. 3A to 3C show the distance "a" between the facing walls, the width "b" and the height "c" of each wall 12, as in Figs. 1 and 2. In this case, it is only necessary that any of the adipocytes forming the cell mass adhere to the culture bottom surface 14 and the same adipocyte or any other adipocytes adhere to the walls 12. In other words, it is only necessary that the cell mass as a whole adhere to the culture bottom surface 14 and the walls.

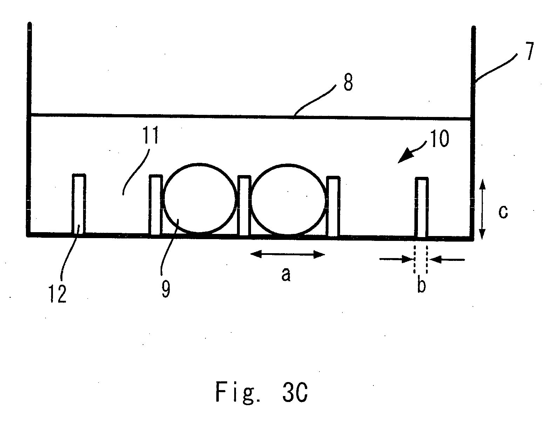

Fig. 3B shows a state where one adipocyte 9 has increased in size in a state where the adipocyte adheres to two locations, i.e., one wall 12 and the culture bottom surface 14, in each region 11 partitioned by the walls 12. Fig. 3C shows a state where an adipocyte has increased in size in a state where the adipocyte adheres to three or more locations on the culture bottom surface 14 and a plurality of walls constituting each culture region. Thus, in the case of culturing one adipocyte in each of the partitioned culture regions, it is only necessary that one adipocyte adhere to two or more locations on the culture bottom surface 14 and the walls 12.

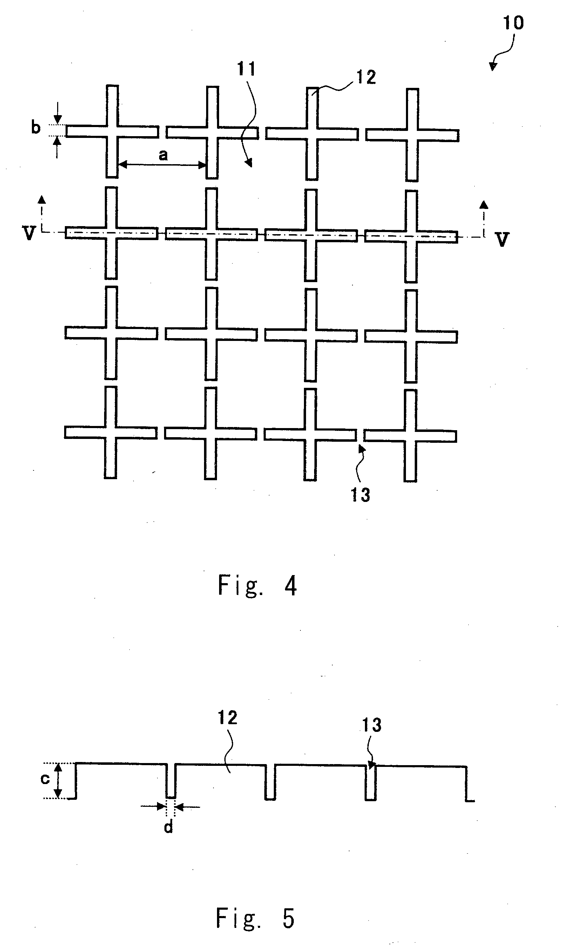

Note that as shown in Figs. 4 and 5, in the partitioned regions 11 partitioned by the walls 12, the walls 12 which are formed perpendicularly to the culture bottom surface 14 may be non-continuous. A width "d" between the non-continuous walls 12 may be such a value at which cultured cells are prevented from moving to the adjacent regions (partitioned regions 11) from the partitioned region 11 in which the cultured cells are originally seeded. For example, when the cultured cells have an equivalent diameter of 20 µm, the width is preferably 5 to 15 µm. Fig. 4 is a plan view showing another structure of the culture chamber according to this embodiment, and Fig. 5 is a sectional view taken along the line V-V of Fig. 4.

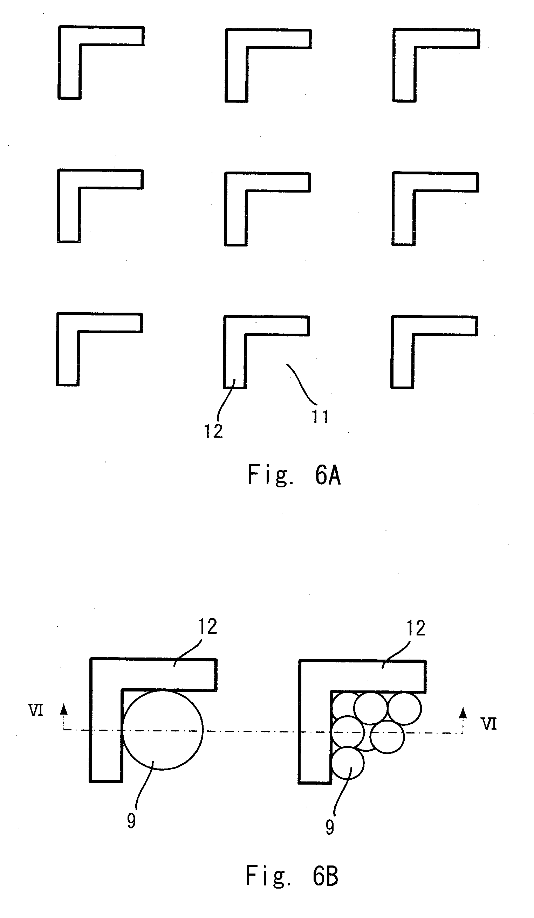

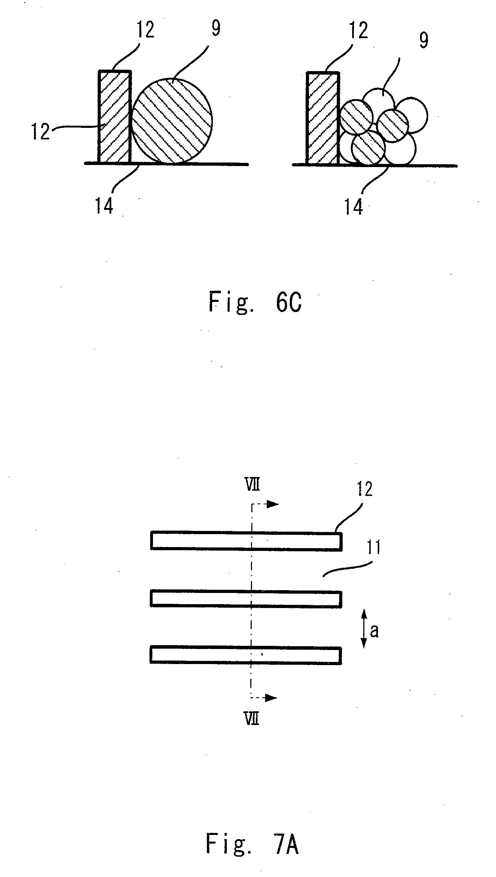

The walls 12 formed on the culture surface may have a shape as shown in Fig. 6A. Fig. 6B is a view showing a state where the adipocytes 9 adhere to the walls 12, when viewed from above. Fig. 6C is a sectional view taken along the line VI-VI of Fig. 6B. Each of Figs. 6B and 6C shows an example in which one adipocyte adheres to the wall 12 on the left side and a cell mass composed of a plurality of adipocytes 9 adheres to the wall 12 on the right side.

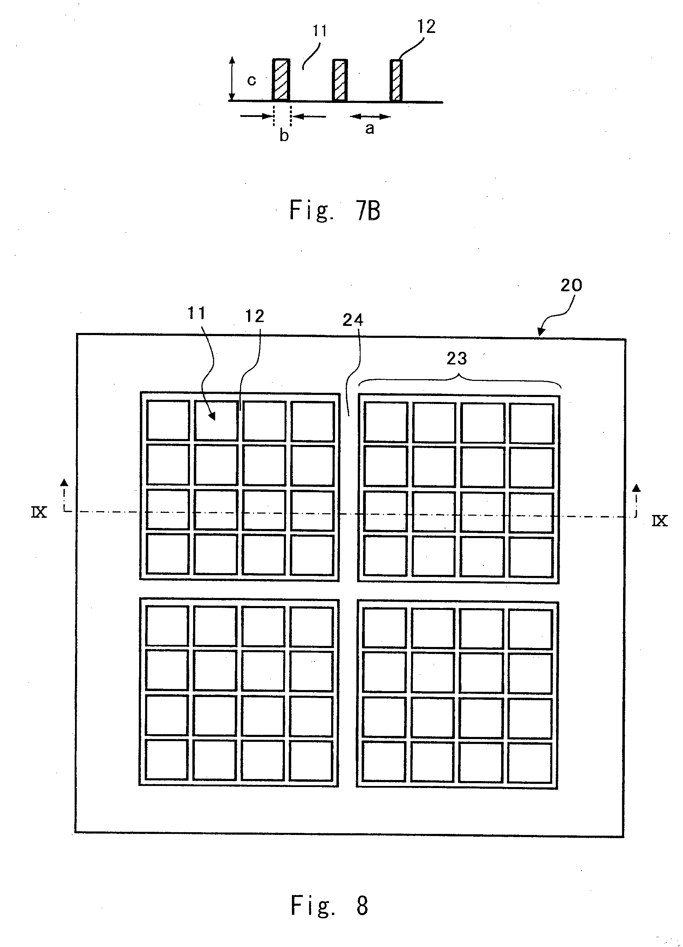

Further, as shown in Figs. 7A and 7B, the walls 12 formed on the culture surface may be formed such that the continuous walls 12 are formed from one end to the other end of the culture surface. Fig. 7B is a sectional view taken along the line VII-VII of Fig. 7A.

The shapes of the walls 12 are not limited to those shown in the drawings. The walls 12 preferably have a shape that allows adipocytes to easily adhere to side walls of the walls 12.

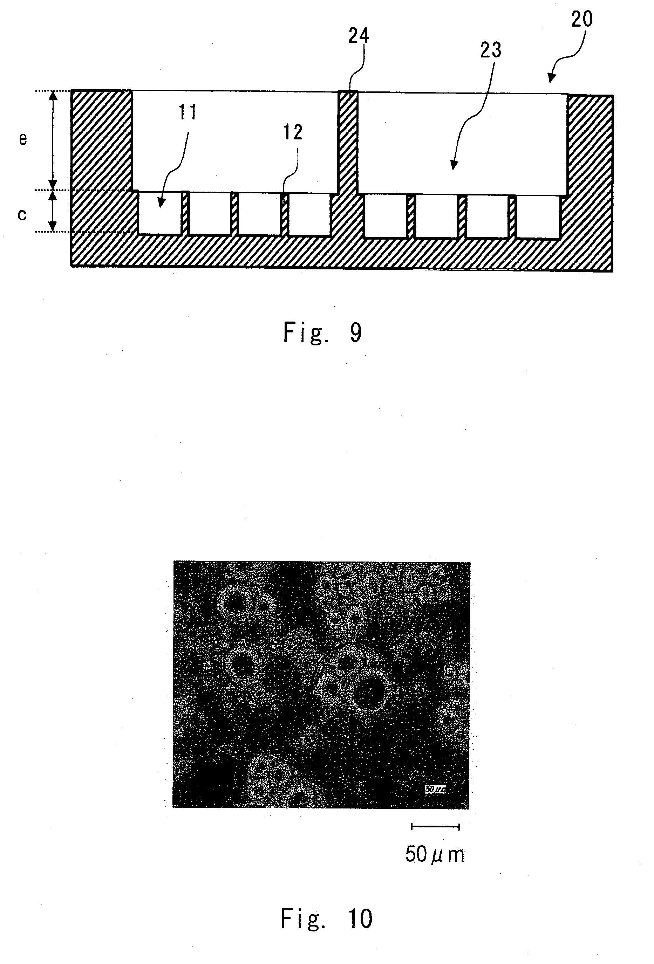

Furthermore, as shown in Figs. 8 and 9, the culture chamber according to this embodiment may include spots having a culture surface with the walls 12 perpendicular to a predetermined number of culture bottom surfaces. Fig. 8 is a plan view showing further another structure of the cell couture portion according to this embodiment. Fig. 9 is a sectional view taken along the line IX-IX of Fig. 8. Each of Figs. 8 and 9 shows an example using the structure of the chamber including the walls perpendicular to the culture bottom surface as shown in Figs. 1 and 2. Fig. 8 shows a plurality of side walls 24 and spots 23 in which the regions 11 partitioned by the walls 12 perpendicular to the culture bottom surface are regularly arranged. A height "e" of each side wall 24 is not particularly limited as long as a sufficient capacity to hold a supernatant fluid, such as a culture solution or a reaction solution, without being dried can be ensured. The height "e" may be set as appropriate. The provision of the side walls 24 enables the use of different culture mediums for each spot 23. While Figs. 8 and 9 show an example of the structure including the side walls 24, a structure with no side walls 24 may also be employed.

A method for preparing the walls perpendicular to the culture bottom surface is not particularly limited, but methods such as transfer molding using a mold, three-dimensional stereolithography, precision machining, wet etching, dry etching, laser processing, and electrical discharge machining may be employed. It is preferable to appropriately select these production methods in view of the intended use, required processing accuracy, costs, and the like of the culture chamber.

As a specific example of the transfer molding method using a mold, a method for forming a concave-convex structure having walls perpendicular to the culture surface, by resin molding using a metal structure as a mold may be employed. This method is preferred because it is capable of reproducing the shape of the metal structure on a resin with a high transcription rate, and because the raw material cost can be reduced by using a general-purpose resin material. Such a method using a mold of a metal structure is superior in terms of low cost and satisfying high dimensional accuracy.

Examples of the method for producing the metal structure include plating treatment on a resist pattern produced by photolithography or a resin pattern produced by three-dimensional stereolithography, precision machining, wet etching, dry etching, laser processing, and electrical discharge machining. The methods may be appropriately selected in view of the intended use, required processing accuracy, costs, and the like.

Examples of the method for forming the concave-convex structure on a resin using the metal structure, which is obtained as described above, as a mold, include injection molding, press molding, monomer casting, solvent casting, hot embossing, or roll transfer by extrusion molding. It is preferable to employ injection molding in view of its productivity and transcription property.

Materials for forming the culture chamber are not particularly limited as long as the materials have self-supporting properties. For example, synthetic resin, silicon, or glass may be employed. A transparent synthetic resin is preferably used as a material in view of costs and cell visibility under microscopic observation. Examples of the transparent synthetic resin include acrylic resins such as polymethylmethacrylate and methyl methacrylate-styrene copolymer, styrene resins such as polystyrene and acrylic styrene copolymer resin, olefin resin such as cycloolefin, ester resins such as polyethylene terephthalate, polylactic acid, and polyglycolic acid, silicone resin such as polydimethylsiloxane, polycarbonate resin, polyester resin, polyvinylalcohol resin, ethylene-vinylalcohol copolymer resin, thermoplastic elastomer, vinyl chloride resin, and silicon resin. These resins may contain various additives such as colorant, dispersing agent, and thickening agent, unless the transparency is impaired.

In the culture chamber, surface treatment may be performed on the culture surface (the culture bottom surface 14, side walls of the walls 12, and upper surfaces of the walls 12) and a modified layer and/or a coating layer having a functional group may be formed for the purpose of improving the hydrophilic properties, biocompatibility, cellular affinity, and the like of the chamber surface. A method for forming the modified layer is not particularly limited unless a method with which the self-supporting properties are impaired and a method causing extreme surface roughness of 100 µm or more are employed. Methods, for example, treatment by chemical reagent, solvent treatment, treatment by chemical reagent such as introduction of a graft polymer by surface graft polymerization, physical treatment such as corona discharge, ozone treatment, or plasma treatment may be employed. In addition, though a method for forming the coating layer is not particularly limited, methods, for example, dry coating such as sputtering or vapor deposition and wet coating such as inorganic material coating or polymer coating may be employed. In order to pour a culture solution, without mixing air bubbles therein, into each boundary portion between the culture bottom surface 14 and the side walls of the walls 12, each space surrounded by the walls 12, and each gap between the adjacent walls 12, it is desirable to impart the hydrophilic properties. As a method for forming a uniform hydrophilic membrane, inorganic vapor deposition is preferably employed.

When the cellular affinity is taken into consideration, it is more preferable to coat cytophilic proteins such as collagen, fibronectin, and laminin. In order to uniformly coat a collagen aqueous solution or the like, it is preferable to perform the coating after the above-mentioned hydrophilic membrane is formed. It is desirable to culture cells on an extracellular matrix surface by replicating the in vivo environment. Accordingly, it is particularly preferable to dispose an organic layer made of an extracellular matrix suitable for cultured cells after an inorganic hydrophilic membrane is uniformly formed as described above.

The above-described surface treatments may be performed singly or appropriately combined as needed.

In addition, it is preferable that the bottom of the culture chamber including the walls perpendicular to the culture bottom surface 14 be transparent. This enables the culture chamber to be directly used in the case of observing cultured adipocytes with a microscope after the adipocytes are cultured. This eliminates the need for transferring the cultured adipocytes to another chamber or the like which enables microscopic observation. Consequently, simplification of a procedure for testing or the like and a reduction in time can be achieved. Moreover, the adipocytes can be prevented from being damaged during the transfer of the cultured adipocytes to another chamber.

### 2. Cell Culture Method

In this embodiment, the shape shown in Fig. 1 described above is used as that of the culture chamber 10 including the walls 12 perpendicular to the culture bottom surface 14. Adipocytes are cultured in a state where the adipocytes are allowed to adhere to the culture bottom surface 14 and the walls 12. By this culture method, mature adipocytes having a three-dimensional shape and containing spherical lipid droplets within cells are obtained. The term "three-dimensional" herein described refers to a shape more similar to a spherical form in which spherical lipid droplets are stored, that is, the shape of each adipocyte in vivo.

The following terms are herein used to explain the cell culture method.

The term "adipocytes" refers to cells containing lipid droplets within the cells. Adipocytes, which are cells derived from animal fibroblasts, differentiate such that fat is stored in the cytoplasm, thereby forming adipose tissue. Adipocytes are divided into unilocular adipocytes (white adipocytes) and multilocular adipocytes (brown adipocytes). Unilocular adipocytes are stored-type cells in which large lipid droplets exist and nuclei and cell organelles are pressed against marginal portions. Multilocular adipocytes are metabotropic cells in which a number of small-sized or middle-sized lipid droplets exist and cell organelles have been developed.

Adipocytes include cells differentiated from bone-marrow stromal cells, mesenchymal stem cells, embryonic stem cells, and induced pluripotent stem cells. The term "bone-marrow stromal cell" herein described refers to tissue which is present in a bone marrow and has a network structure. The term "mesenchymal stem cell" refers to a stem cell which is present in mesenchymal tissue, such as osteocytes and adipocytes, and which can differentiate into mesenchyme (adipocyte, bone, etc.) cells. Mesenchymal stem cells can be separated from a bone marrow or adipose tissue. The mesenchymal stem cells are stem cell lines formed of an inner cell mass belonging to a part of a blastocyst-stage embryo which is the generation initial phase of an animal. Outside a living organism, the mesenchymal stem cells enable substantially infinite proliferation while maintaining the pluripotent differentiation into any type of tissue in theory. The induced pluripotent stem cells are a type of embryo-stem cells and enable proliferation in the same manner as embryonic stem cells and differentiation into various types of cells. The iPS cells can be produced from skin cells and the like.

The term "lipid droplets" refers to droplets of fat present in the cytoplasm, which are observed in the process of differentiation into adipocytes, for example. As the cells mature, these droplets are combined and large spherical lipid droplets are gradually formed.

The term "mature adipocyte" refers to an adipocyte in which a lipid droplet is stored. The size of each lipid droplet of mature adipocytes is not particularly limited as long as lipid droplets are formed in the cells. Therefore, the mature adipocytes include an adipocyte containing a lipid droplet having increased in size and an adipocyte containing a lipid droplet smaller than the lipid droplet having increased in size. For example, the term "minute lipid droplet" herein described refers to a lipid droplet having a size less than 0.5×10³ µm³, and also refers to an adipocyte obtained before the adipocyte has increased in size and finally differentiated into a mature cell from a preadipocyte. Herein, adipocytes to be cultured include mature adipocytes in which minute lipid droplets are stored. The mature adipocytes in which the minute lipid droplets are stored are cultured to obtain mature adipocytes in which lipid droplets having increased in size are stored.

The term "differentiate" refers to the production of a cell type that is more differentiated than the cell type from which it is derived. The term therefore encompasses cell types that are partially and terminally differentiated.

The term "differentiation induction" refers to a phenomenon in which a specific type of tissue or chemical substance naturally or artificially causes differentiation of another tissue or cell in the development and differentiation of a living organism. The substance that causes the differentiation induction is referred to as "differentiation inducing substance".

The term "adherent culture" refers to culture of cells while allowing the cells to adhere to the culture chamber.

The term "subculture" refers to cell culture in which a part of cells is transferred to a new culture chamber and the cells are cultured again.

The cell culture method uses a culture surface including the walls 12 perpendicular to the culture bottom surface 14, for example, a culture surface which has a three-dimensional shape and includes the regions 11 partitioned by the walls 12 as described above. Adipocytes are cultured in a state where the adipocytes are allowed to adhere to the culture bottom surface 14 and the side walls of the walls 12. This prevents the cells from being peeled off from the culture surface and floating, even when the differentiation of the cells has advanced and the lipid droplets have increased in size. In addition, the cells are formed into a three-dimensional shape. In this manner, the cells are cultured while the cells are allowed to adhere to the partitioned regions 11, thereby obtaining mature adipocytes which have a three-dimensional shape and contain spherical lipid droplets within cells.

This cell culture method makes it possible to culture cells on a well plate, for example, without the need of culturing cells while allowing the cells to adhere to the ceiling surface of the culture chamber (flask) as disclosed in Patent Literature 2, for example.

In the cell culture method of this embodiment, the adipocytes (mature adipocytes), the steps of the cell culture method, and the like may have the following features.

The mature adipocytes having increased in size, which are obtained by culture, preferably contain spherical lipid droplets having a diameter of 20 µm to 180 µm. That is, the volume of the lipid droplets is preferably in the range from 4×10³ µm³ to 3×10⁶ µm³, and a value (calculated value) obtained by dividing a minimum diameter (minor axis) of the lipid droplets by a maximum diameter (major axis) thereof is preferably in the range from 0.4 to 1.0.

The cell culture method may include a structure for differentiation induction that is carried out after the adipocytes are proliferated in the culture chamber including the walls 12 perpendicular to the culture bottom surface 14. The term "structure for differentiation induction" refers to a process of culturing cells by changing a culture medium used for proliferation with a culture medium including a differentiation inducing factor. Specifically, the method includes a step (substance) for culture in a culture medium including insulin.

When the adipocytes to be cultured are formed in two or more layers, specifically, when the adipocytes fully cover the culture bottom surface 14, so that there is no space for proliferation and the adipocytes are formed in layers, the differentiation induction is started from a 90% or more confluent state.

A cell seeding density of the cells is not particularly limited as long as the cells can survive and proliferate. It is preferable to carry out a differentiation induction step after cells are seeded at a density of 0.1×10⁴/cm² to 10×10⁴/cm² and the cells are proliferated to a 90% confluent state and differentiation-induced using a differentiation-inducing culture medium. The cells which are differentiating but do not proliferate are preferably seeded at a density of 10×10⁴/cm² to 1×10⁶/cm²_{.}

By the cell culture method described above, mature cells which have a three-dimensional shape and contain spherical lipid droplets within cells are obtained. This achieves a state similar to the in vivo environment in which each adipocyte exists as a sphere and the spheres aggregate. This makes it possible to provide adipocytes having a shape similar to that in vivo. Furthermore, the use of the culture chamber 10 including the walls 12 perpendicular to the culture bottom surface 14 as described above makes it possible to provide a method that enables culture of mature adipocytes by use of a chamber having a shape similar to that of a well plate, a petri dish, and the like, which are generally used, without the need for complicated operation such as ceiling culture or gel culture which is culture within a gel. In addition, the culture method makes it possible to provide a group of mature adipocytes suitable for drug screening, for example.

Moreover, it is possible to provide a method that enables culture of mature adipocytes that secrete a large amount of adiponectin which is one of cytokines specifically produced by adipocytes.

### [Examples]

### [Example 1-3, Comparative Example 1]

### 1. Culture chamber

As the culture surface on which the walls perpendicular to the culture bottom surface are formed, a film of 1 cm × 1 cm having the culture surface shown in Figs. 1 and 2 described above was attached to the bottom surface of the culture chamber having a well-plate shape, and established preadipocytes (3T3-L1) were cultured.

This film portion was used as the culture surface in Examples, and a flat portion excluding the film was used as the culture surface in Comparative Example.

### 1-1. Examples 1 and 2

As culture surfaces for use in Examples 1 and 2, which include the walls 12 perpendicular to the culture bottom surface 14, the following two types of films having the partitioned regions 11 were prepared. In Example 1, the distance "a" shown in Fig. 1 is 200 µm and the height "c" shown in Fig. 2 is 50 µm. In Example 2, the distance "a" shown in Fig. 1 is 200 µm and the height "c" shown in Fig. 2 is 100 µm.

### 1-2. Comparative Example 1

In Comparative Example 1, a flat portion excluding the film of 1 cm × 1 cm was used as the culture surface.

### 2. Cell culture and observation

### 2-1. Examples 1 and 2

The established preadipocytes (3T3-L1) were subjected to subculture in a 25 cm² flask, and cells were seeded in each well when a 70% confluent state was reached.

The term "confluent" herein described refers to a ratio of an area occupied by cells to the entire culture surface. For example, in the case of using a flak having an area of 25 cm², a state in which 50% of the area is occupied by cells is referred to as "50% confluent". The term "70% confluent" refers to a state in which 70% of the entire culture surface is covered with cells. When term "confluent state" herein described with no values given indicates a 90% or more confluent state.

Cells were seeded at a density of 0.145 to 1.16×10⁴/cm².

As a culture solution, D-MEM (Gibco-Invitrogen, #11965-084 or #12100-046) + 10% Donor Calf Serum (Tissue Culture Biologicals, #301D) + Antibiotic-Antimycotic (Gibco-Invitrogen, #15240-062) was used until the confluent state was reached, and the culture solution was changed once every two days.

After the cells reached the confluent state, the culture medium was replaced with a differentiation-inducing culture medium, and the differentiation induction was started. Assume that the day on which the differentiation induction was started is the 0th day of the differentiation induction. As the differentiation-inducing culture medium, D-MEM + 10% Fetal Bovine Serum (Bio West, #S1820) + Antibiotic-Antimycotic + 0.25 µM Dexamethasone (Nacalai, # 11107-51) + 0.5 mM 3-Isobutyl-1-Metylxanthine (Nacalai, #19624-31) + 4 µg/ml Humulin R (Eli Lilly Japan, 100 U/ml, #HI0210) was used during the period from the 0th day to the 2nd day of the differentiation induction. During the period from the 2nd day to the 4th day of the differentiation induction, D-MEM + 10% Fetal Bovine Serum + Antibiotic-Antimycotic + 4 µg/ml Humulin R was used. On and after the 4th day of the differentiation induction, D-MEM + 10% Fetal Bovine Serum + Antibiotic-Antimycotic was used.

After the cells were seeded in each well, the cells were differentiated and cultured for 20 days and observed with an inverted microscope. In Example 2, in addition to the observation with an inverted microscope, fluorescence staining of lipid droplets was carried out using 1 µg/ml Hoechst 33342, while maintaining them alive without fixing the nuclei, on the 31st day of the differentiation induction. A slice image sequence in a Z-axis (height) direction of the fluorescence-stained cells was captured by a confocal scanning laser microscope (slice image sequence of cell staining).

The fluorescence staining used in cell staining has the following features. When light is applied to a fluorescent dye, electrons are excited. However, the electrons return to a stable place. At the time of return, the electrons return while releasing energy, which causes an energy loss. Accordingly, the released energy is released as energy smaller than that obtained from light. Bodipy 493/503 is a fluorescent dye which is selectively dissolved in neutral fat, and absorbs blue light and emits green light. Hoechst 33342 is a fluorescent dye that is selectively bound to DNA, and absorbs violet (UV) light and emits blue light. In the captured black and white images, a green portion appears in white.

### 2-1. Comparative Example 1

A flat portion of Examples 1 and 2 was used. Accordingly, the conditions are the same except that the culture bottom surface is different.

On the 20th day of the differentiation induction after the cells were seeded, the cells were observed with an inverted microscope in the same manner as in Examples 1 and 2.

### 3. Results

### 3-1. Cells obtained after 20 days of differentiation

The results of the cultures under the respective conditions of Example 1-3 and Comparative Example 1 will be described with reference to photographs of cells obtained after 20 days of the differentiation.

### -Comparative Example 1

Fig. 10 shows a photograph obtained by capturing an image of cells after 20 days of the differentiation in Comparative Example 1. In Fig. 10, an encircled portion corresponds to one cell. In Fig. 10, the size of each lipid droplet of the encircled cell was about 50 µm. The cell contained a plurality of large lipid droplets, so the cell was not spherical.

### -Example 1

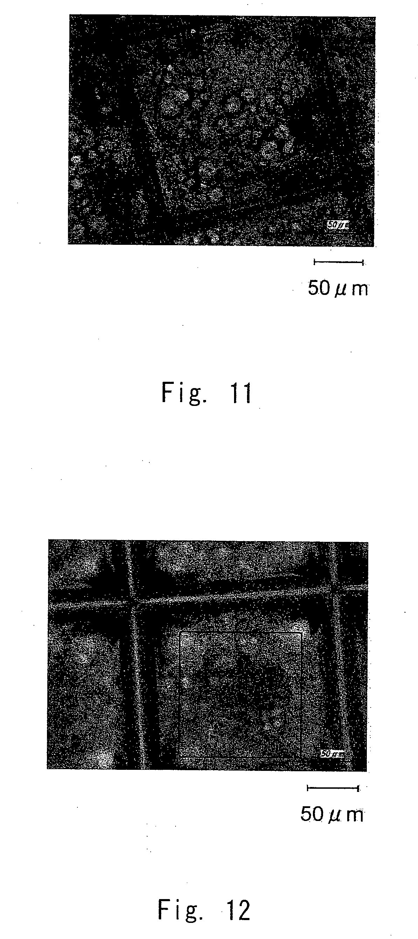

Fig. 11 shows a photograph obtained by capturing an image of cells after 20 days of the differentiation in Example 1. In Fig. 11, an encircled portion corresponds to one cell. A portion surrounded by a square is an aggregate of cells. After 20 days of the differentiation, an aggregate of cells in the vicinity of the center of the partitioned region was observed.

In the observation before the lapse of 20 days of the differentiation, cells were present not only in the vicinity of the center of the microchamber, but also in the vicinity of the walls (side walls), and covered the bottom. However, as the cells matured, the cells aggregated in the vicinity of the center of the microchamber. A 40x photograph of Fig. 11 shows that lipid droplets overlap with each other. Accordingly, a state was observed in which cells aggregate in the vicinity of the center and each of the cells forms a mass. From this observation, it is estimated that the cells form a cell aggregate in a self-assembled manner.

In addition, the size of each cell tends to be smaller than that of Comparative Example 1. It is estimated that this is not because the volume of each cell is small, but because the form of each cell is not flat but spherical in Example 1.

### -Example 2

Fig. 12 shows a photograph obtained by capturing an image of cells after 20 days of the differentiation in Example 2. In Fig. 12, a portion surrounded by a square shows that cells aggregated in the vicinity of the center of the microchamber and formed a mass of adipocytes.

### 3-2. Slice image of cell staining

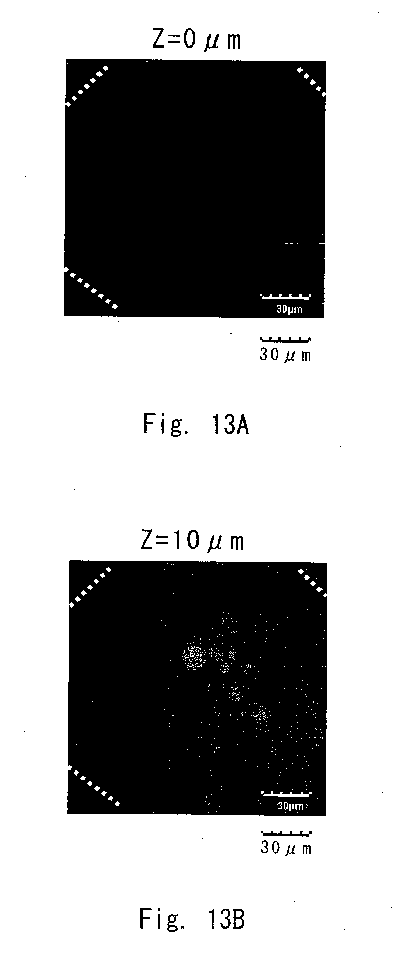

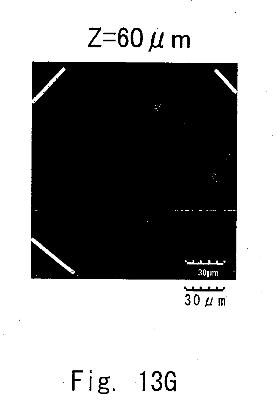

Figs. 13A to 13G show photographs of a slice image sequence captured after cell staining in Example 2. A scale shown in a lower right portion of the photograph indicates a length of 30 µm. Stained cells were photographed from above, while an X-axis and a Y-axis were fixed and the cells were moved in a Z-axis direction. Fig. 13A is a photograph showing cells in a bottom portion. Cells were photographed sequentially toward the upper side. Fig. 13G shows cells in an upper portion. In Figs. 13A to 13G, portions appearing in white correspond to lipid droplets. In Figs. 13A to 13G, dotted oblique lines shown in upper-left, lower-left, and upper-right portions indicate the walls of the microchamber.

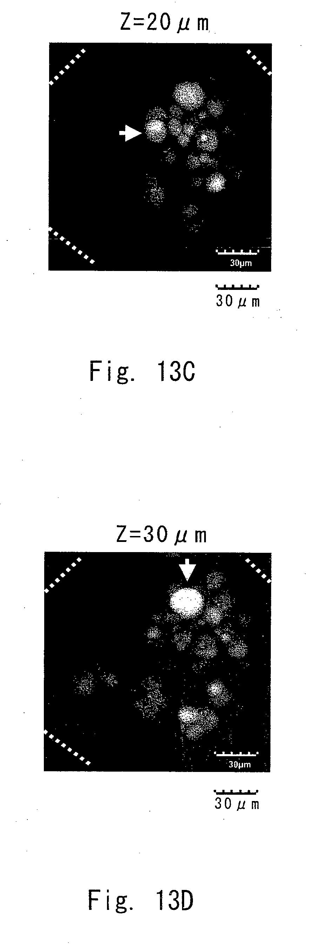

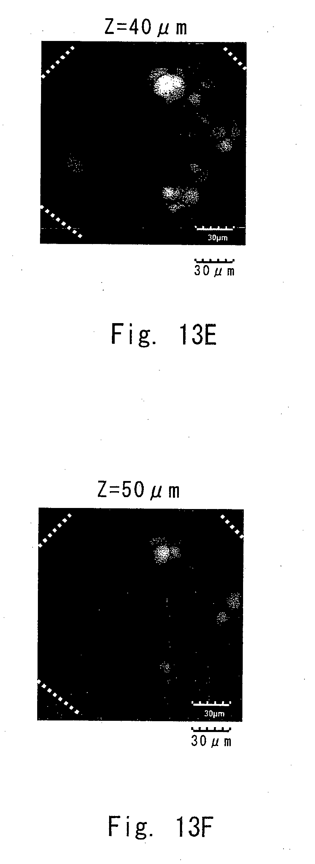

It is observed that the cytoplasm of a cell indicated by an arrow in Fig. 13C (Z=20 µm) is occupied by one lipid droplet, and the cell has a diameter of 20 µm in an X-Y plane. On the other hand, in the observation in the Z-axis direction, the cell is clearly observed in Fig. 13B (Z=10 µm) to Fig. 13D (Z=30 µm), whereas the cell appears blurred in Fig. 13A (Z=0 µm) and Fig. 13E (Z=40 µm).

It is observed that the cytoplasm of a cell indicated by an arrow in Fig. 13D (Z=30 µm) is also occupied by one lipid droplet, and the cell has a diameter of 30 µm in the X-Y plane. On the other hand, in the observation in the Z-axis direction, the cell is clearly observed in Fig. 13C (Z=20 µm) to Fig. 13E (Z=40 µm), whereas the cell appears blurred in Fig. 13A (Z=0 µm) and Fig. 13F (Z=50 µm).

It can be estimated that each cell has a diameter of 20 to 40 µm in the Z-axis direction. Since the diameter is in the range from 20 to 30 µm as described above, the lipid droplets of the cells forming a cell aggregate in Example 2 have a spherical shape as a cell form.

This application is based upon and claims the benefit of priority from Japanese patent application No. 2011-057770, filed on March 16, 2011, the disclosure of which is incorporated herein in its entirety by reference.

### Reference Signs List

- 7: PETRI DISH OR WELL PLATE
- 8: CULTURE MEDIUM
- 9: CELL
- 10: CULTURE CHAMBER
- 11: PARTITIONED REGION
- 12: WALL
- 13: OPENING
- 14: CULTURE BOTTOM SURFACE
- 23: SPOT
- 24: SIDE WALL OF SPOT

## Claims

1. A culture method for adipocytes which uses a culture chamber including a culture bottom surface and walls disposed perpendicularly to the culture bottom surface, to obtain spherical mature adipocytes in which spherical lipid droplets having increased in size are stored, by allowing cultured adipocytes to adhere to the culture bottom surface and the walls, without suspending the adipocytes in a culture solution.

2. The culture method according to Claim 1, wherein the cultured adipocytes contain lipid droplets of less than 0.5×10³ µm³.

3. The culture method according to Claim 1, wherein the cultured adipocytes are adipocytes differentiated from bone-marrow stromal cells, mesenchymal stem cells, embryonic stem cells, or induced pluripotent stem cells.

4. The culture method according to Claim 1, wherein the cultured adipocytes are preadipocytes.

5. The culture method according to Claim 4, comprising a step of differentiation-inducing the preadipocytes after the preadipocytes are proliferated to a 90% confluent state.

6. The culture method according to Claim 5, wherein the differentiation induction step includes the steps of: (1) culturing the preadipocytes in a culture medium including dexamethasone and insulin; (2) culturing the preadipocytes in a culture medium including insulin but not including dexamethasone; and (3) culturing the preadipocytes in a culture medium including neither insulin nor dexamethasone.

7. The culture method according to any one of Claims 1 to 6, comprising a step of seeding cells at a cell seeding density of 0.1×10⁴/cm² to 1×10⁶/cm² in the culture chamber.

8. The culture method according to any one of Claims 1 to 7, wherein in the culture chamber, the walls disposed perpendicularly to the culture bottom surface have a height of 10 µm to 500 µm.

9. The culture method according to any one of Claims 1 to 8, wherein in the culture chamber, the walls disposed perpendicularly to the culture bottom surface are continuous.

10. The culture method according to any one of Claims 1 to 8, wherein in the culture chamber, the walls disposed perpendicularly to the culture bottom surface are not continuous.

11. The culture method according to any one of Claims 1 to 10, wherein in the culture chamber, regions partitioned by the walls disposed perpendicularly to the culture bottom surface are formed.

12. The culture method according to Claim 11, wherein the partitioned regions are regularly arranged.

13. The culture method according to any one of Claims 1 to 12, wherein in the culture chamber, a shortest distance between an arbitrary one of the walls disposed perpendicularly to the culture bottom surface and another wall facing the one wall is equal to or more than 10 µm.

14. The culture method according to Claim 11 or 12, wherein an inscribed circle diameter of a bottom area of each of the partitioned regions is in a range from 0.1 times to 4 times larger than a height of a side wall.

15. The culture method according to any one of Claims 1 to 14, wherein the spherical lipid droplets having increased in size have a volume of 0.5×10³ µm³ to 3×10⁶ µm³

16. The culture method according to any one of Claims 1 to 15, wherein the adipocytes have such a shape that a value obtained by dividing a minimum diameter (minor axis) of the lipid droplets by a maximum diameter (major axis) thereof is 0.4 to 1.0.

17. The culture method according to any one of Claims 1 to 16, wherein a bottom of the culture chamber is transparent.

18. The culture method according to any one of Claims 1 to 16, wherein the culture chamber includes a culture bottom surface having a polar functional group and walls disposed perpendicularly to the culture bottom surface.

19. The culture method according to Claim 17, wherein in the culture chamber, the culture bottom surface and surfaces of the walls disposed perpendicularly to the culture bottom surface are coated with an inorganic material.

20. The culture method according to any one of Claims 17 to 19, wherein in the culture chamber, the culture bottom surface and surfaces of the walls disposed perpendicularly to the culture bottom surface are coated with an extracellular matrix as typified by collagen and fibronectin.

21. The culture method according to any one of Claims 17 to 19, wherein in the culture chamber, the culture bottom surface and surfaces of the walls disposed perpendicularly to the culture bottom surface are coated with a synthetic material.

22. A group of mature adipocytes including a plurality of mature adipocytes cultured in a culture chamber including a culture bottom surface and walls disposed perpendicularly to the culture bottom surface, by allowing the adipocytes to adhere to the culture bottom surface and the walls, without suspending the adipocytes in a culture solution, spherical lipid droplets having increased in size having a volume of 0.5×10³ µm³ to 3×10⁶ µm³ being stored in the mature adipocytes.

23. A drug screening method for examining an effect of a drug on adipose tissue by allowing the drug to act on mature adipocytes of the group of mature adipocytes according to Claim 21.
